(19) Europäisches Patentamt

European Patent Office

Office européen des brevets

(11) Publication number: **0 503 517 A1**

## EUROPEAN PATENT APPLICATION

(21) Application number: **92103948.3**

(22) Date of filing: **07.03.92**

(30) Priority: **11.03.91 US 667121**

(43) Date of publication of application:
**16.09.92 Bulletin 92/38**

(84) Designated Contracting States:
**DE DK GB SE**

(51) Int. Cl.⁵: **G01N 33/569**, G01N 33/543

(71) Applicant: **Becton Dickinson and Company
One Becton Drive
Franklin Lakes New Jersey 07417-1880(US)**

(72) Inventor: **Naqui, Ali
610 Cargill Court
Belaire Maryland 21015(US)**
Inventor: **Mapes, James P.
8005 Kingsland Drive
Raleigh, North Carolina 27613(US)**
Inventor: **Keating, William E.
605 Jones Ferry Road, No. SS-10
Carrboro, North Carolina 27510(US)**

(74) Representative: **von Kreisler, Alek,
Dipl.-Chem. et al
Patentanwälte Von Kreisler-Selting-Werner,
Deichmannhaus am Hauptbahnhof
W-5000 Köln 1(DE)**

(54) Improved assay for lyme disease.

㊗ Diagnosis of Lyme disease by immunoassay for anti-Borrelia burgdorferi antibodies in a patient sample includes a denatured Borrelia burgdorferi capture antigen adsorbed onto a solid support. The preferred assay is a flow-through assay using a porous membrane as the support and a dye-loaded liposome conjugated to a goat antihuman immunoglobulin for detection. The invention includes a kit of materials for performing the assay.

## BACKGROUND OF THE INVENTION

1. Field of the Invention. This invention relates to Lyme disease, and more specifically relates to an assay for serum antibodies against Lyme disease antigen in which crossreactivity of the antigen to other antibodies in the serum is reduced.

2. Background of the Invention. Borrelia burgdorferi, a species within the genus Borrelia and family Treponemataceae is a tick-borne spirochete which has been identified as the causative agent of Lyme disease.

Several approaches to diagnosis of Lyme disease have been investigated. Diagnosis by histological identification of the spirochete is very difficult because the spirochete is present in tissue or body fluid in small numbers, even in many advanced cases, and isolation is even more difficult. Diagnosis by assay for Borrelia antigen is disclosed in U.S. Patent No. 4,888,276 to Shelbourne. In the Shelbourne assay, a urine sample from a patient suspected of having Lyme disease is combined with an anti-Borrelia antibody, monoclonal or polyclonal, raised against the antigen and the antigen-antibody complex is detected with an enzyme labeled anti-antibody.

Most reports on diagnosis of the disease have relied on assay of serum samples for anti-Borrelia antibodies present in a patient's serum in response to infection. Several types of assays have been developed including enzyme linked immunosorbent assay (ELISA) and immunofluorescence assay (IFA).

At the present state of the art, ELISA and IFA assays of sera from patients in the early stages of the disease, some of whom may be clinically asymptomatic, is generally recognized to be unsatisfactory because of weak antibody response. Also, false positives arising from crossreactivity with other antibodies in the serum decrease the specificity of the assays. For example, Russell et al. in the Journal of Infectious Diseases, 149, 465 (1984) assayed sera from healthy individuals, patients with Lyme disease, and patients with other infections by ELISA and IFA and found significant crossreactivity unless sera from other treponemal-infected patients were excluded.

Likewise, Grodzicki et al. in the Journal of Infectious Diseases, 157, 790 (1988) describes a comparative study of the effectiveness of an indirect ELISA assay and an immunoblot assay for diagnosis of early Lyme disease, and concludes that less false positives result with the immunoblot method.

Attempts to improve assays for Lyme disease by enrichment of Borrelia antigen or adsorption of the crossreacting antibodies with proteins have been reported. Thus, Coleman et al., in the Journal of Infectious Diseases, 155, 756 (1987) shows that assay for Lyme disease by ELISA is improved by removal of the outer envelope fraction of the spirochete, electrophoresis and Western blotting of the residue to isolate a flagellin-enriched protein fraction and use of this material as the capture antigen. Fawcett et al. adsorbs crossreacting antibodies with E. coli to reduce nonspecific binding and false positives.

Raoult et al., in the Journal of Clinical Microbiology, 27, 2152 (1989) discloses reduction in crossreactivity of sera positive for leptospirosis syphilis or human immunodeficiency virus in the micro IFA test. The Raoult et al. method includes adsorption of crossreacting antibodies with an ultrasonicate of Reiter trepenomes at 37°C for 60 minutes. On the other hand, Magnarelli et al. in the American Journal of Epidemiology, 127, 818, (1988) report that, to date there has been limited success in efforts to increase the specificity of ELISA for Lyme disease by adsorption with Borrelia hermsii or Reiter trepenomes.

Craft et al., in the Journal of Infectious Diseases, 149, 789 (1984) compares ELISA and IFA assays which include 90 minute preadsorption at 23°C. Craft et al. concludes that ELISA is more sensitive and specific than IFA, and ELISA without preadsorption of crossreacting antibodies is the best diagnostic test of Lyme disease.

A commercial Lyme disease assay kit sold by Zeus Scientific Inc. Raritan, New Jersey, utilizes the IFA technique and is claimed to be useful for confirmation of Lyme disease in its later stages. A commercial ELISA kit sold under the trade name IMMUNOCLONE™ by Access Medical Systems is claimed to be more accurate than the leading commercially available laboratory methods.

In a copending application of common assignee herewith, a method of assay for Lyme disease is disclosed in which crossreactive antibodies are preadsorbed by treating a serum sample with particular microorganisms.

Lyme disease was first identified in 1975, and today is well-recognized as a growing menace. In spite of extensive research, no satisfactory assay for the disease has yet been advanced, and diagnosis still relies heavily on clinical observations. There is thus a need for a rapid and reliable assay of high sensitivity and specificity. The present invention is directed to fulfilling this need.

## SUMMARY OF THE INVENTION

An assay for Lyme disease includes binding of anti Borrelia burgdorferi antibodies (hereinafter referred to as analyte antibodies) to denatured Borrelia burgdorferi antigen. For clarity, the denatured antigen of the invention will hereinafter be referred to as the capture antigen and anti Borrelia burgdorferi antigen which has not been denatured will be referred to as native antigen. The term denatured is intended to mean a nonproteolytic change in the antigen structure which causes it to lose some or all of its unique or specific characteristics.

A serum sample from a patient suspected of having Lyme disease is added to a solid support coated with capture antigen to cause binding between capture antigen and analyte antibodies. Other antibodies in the serum (hereinafter called crossreactive antibodies) do not bind to any substantial degree to the capture antigen. The solid support having analyte antibodies captured thereon is contacted with a tracer including a detection antibody labeled with a dye. Binding of the detection antibody to the analyte antibody results in adherence of the dye to the solid support. Detection of the dye is a positive assay for Lyme disease.

The capture antigen may be prepared by treating Borrelia burgdorferi organisms with heat and/or preferably with a denaturant such as sodium dodecyl sulfate (SDS), preferably after sonication.

In a preferred assay, the solid support is a porous membrane coated with capture antigen and the assay is performed by flow through assay in which liquids used as vehicles for the assay reagents or for wash steps pass through the membrane by capillary action induced by adsorbent material under the membrane.

In the most preferred assay, the tracer includes an adsorbing dye encapsulated in a liposome conjugated to an antihuman immunoglobulin detection antibody.

The invention includes a kit of materials for performing the assay.

Thus, the invention provides a simple method to improve Lyme assay specificity. By denaturing the native Borrelia burgdorferi antigen conventionally used for capture, nonspecific binding between the capture antigen and other antibodies in the serum sample, in particular antibodies in the serum recognizing organisms related to Borrelia burgdorferi, is substantially eliminated. The improved method does not require the time consuming, labor intensive and expensive isolation of individual Borrelia burgdorferi capture proteins characteristic of prior art attempts to improve specificity using native antigen or a fragment thereof. Further, the improved assay does not suffer a loss of sensitivity because the liposome carries a multiplicity of dye molecules for each detection antibody and thus provides many labeled detection molecules for each analyte antibody which is captured by the capture antigen. A detectable level of color is thereby achieved even when the serum has a low analyte-antibody titer.

The assay of the invention requires only 90 seconds to complete and has no time-critical steps as compared to 6.5 minutes and three time-critical steps for IMMUNOCLONE™. Accordingly, many more assays may be performed in a short time by relatively unskilled technicians in a laboratory setting, a physician's office or even in the home.

DETAILED DESCRIPTION

While this invention is satisfied by embodiments in many different forms, there will herein be described in detail preferred embodiments of the invention, with the understanding that the present disclosure is to be considered as exemplary of the principles of the invention and is not intended to limit the invention to the embodiments described. The scope of the invention will be measured by the appended claims and their equivalents.

One aspect of the invention is an improved assay for detection of Lyme disease. The assay detects the presence of analyte antibodies to the spirochete, Borrelia burgdorferi, in a patient's serum. In accordance with the invention, crossreactive antibodies in the patient's serum are substantially precluded from binding by denaturing the capture antigen.

The preferred assay of the invention is a solid phase assay in which the capture antigen is adsorbed on a solid support. Any conventional solid support as known in the art may be used, as, for example, the wells of a microtiter plate, a dipstick or the inside wall of a tube or cuvette. The preferred assay technique is flow-through assay in which the solid support is a porous membrane. The membrane may be positioned in any suitable assay device adapted for flow-through assay as known in the art. In preferred devices, flow of assay liquids is promoted by capillary action induced by a pad of adsorbent material adjacent the membrane, and the membrane and adsorbent pad are mounted in a suitable housing. Membrane flow-through assay and various devices therefor have been disclosed and several devices are commercially available.

The porous membrane may be of any material which does not interfere in any way with any other component or step of the assay. Suitable membranes are, for example, of glass fiber, polyvinylidene difluoride, polycarbonate, nitrocellulose and nylon. Such membranes are well-known in the art and many are commercially available from suppliers such as Pall (East Hills, New York), Millipore (Bedford, Massachusetts) and Schleicher and Schuell (Keene, New Hampshire).

The capture antigen may be prepared by denaturation of the native antigen by any procedure as known in the art. In one suitable procedure, the spirochete, available from the American Type Culture Collection, Rockville, Maryland, may be grown in a suitable medium, harvested, and suspended in a suitable diluent such as phosphate buffered saline (PBS). The suspension of cells may be heated for a suitable time such as about 1 minute to 1 hour at a temperature of about 50 to 100°C to effect denaturation. Preferably, the cells may be broken apart by sonication to expose the antigens prior to heat denaturation.

A preferred capture antigen may be obtained by treatment of the native antigen with a denaturant. Suitable denaturants are, for example, zwitterionic detergents, cationic detergents such as cetylpyridinium chloride or cetyltrimethylammonium bromide and preferably anionic detergents. Suitable anionic detergents are sodium cholate, sodium deoxycholate and sodium taurocholate. A preferred anionic denaturant is SDS. The most preferred denaturant for the present invention is a buffered composition including SDS, mercaptoethanol and glycerol. The SDS may be from about 1 to 10, preferably about 2 to 5 weight percent of the composition. The mercaptoethanol may be about 0 to 10, preferably about 3 to 6 weight percent of the composition. The glycerol may be about 0 to 20, preferably about 5 to 10 weight percent of the composition. Suitable buffers are, for example, Tris and phosphate buffered saline (PBS).

Absorption of the capture antigen onto the membrane is wholly conventional and may be performed by covalent attachment or preferably by physical adsorption. In the latter technique, a suitable quantity of capture antigen stock, generally about 300 $\mu$l diluted in a buffer, may be spotted on the membrane and the liquid phase allowed to pass through by capillary action. As the liquid flows through, the antigen is adsorbed on the membrane.

After adsorption of the capture antigen, the membrane may be further coated with an inert protein to fill any binding sites on the membrane not occupied by the capture antigen. (In the present disclosure, the term inert protein means a protein which is immunologically unreactive toward any other component of the assay and which does not substantially bind nonspecifically to other proteins in the assay medium, with the understanding that the inert protein may well be immunologically reactive toward other materials which are not part of the assay of the invention.) Representative nonlimiting examples of suitable inert proteins are casein and albumin, although others will be evident to those skilled in the art.

Serum, plasma or whole blood from a patient suspected of having Lyme disease may then be passed through the membrane. Analyte antibodies present in the serum bind to the capture antigen on the membrane. Crossreactive antibodies in the serum, on the other hand, do not bind and pass through the membrane.

Analyte antibodies captured on the membrane are detected by passing a tracer containing a label through the membrane. A preferred tracer is a dye-labeled detection antibody which binds with the analyte antibody. In the most preferred tracer, the detection antibody is a goat anti-human antibody raised against human immunoglobulin by wholly conventional procedures.

Suitable dyes are adsorbing dyes or fluorescent dyes, and the invention contemplates any dye known in the art as a label in immunoassay. Representative nonlimiting dyes are fluorescein, alizarin red, congo red, brilliant green, toluidine blue and the like. The preferred dye is sulforhodamine B.

In a preferred tracer, the dye is occluded in a particulate medium. As known in the art, the occluding medium may be a polymeric microparticle or it may be any one of the wide variety of sacs known in the art. A particularly useful type of sac is a vesicle, most preferably a liposome. Liposomes may be prepared by any one of a wide variety of procedures. Thus, for example, a liposome may be prepared by a reverse emulsion technique wherein there is provided a water-in-oil emulsion containing the materials for forming the liposome (generally phospholipids), as well as the dye to be occluded. Evaporation of the solvent gives a gel-like mixture which is converted to the liposome having the dye occluded therein by agitation, sonication or addition of the gel-like mixture to water.

In accordance with the invention, the liposome may be of any shape, but preferably is substantially spherical. The preferred liposomes may be from about 0.01 to 1 $\mu$m in diameter, most preferably from about 0.2 to 0.4 $\mu$m. Liposomes within the desired size range may conveniently be obtained by passage of liposomes of mixed sizes through filters of appropriate pore size.

The liposome may be conjugated to the detection antibody by a variety of conventional procedures, preferably by covalent coupling. Covalent coupling may be performed directly or by way of a spacer compound having two reactive functional groups, one of which is capable of reacting or being linked to a functional group of the detection antibody portion of the tracer, and the other of which is capable of reacting or being linked to a functional group on the liposome. In another technique, the detection antibody may be

4

coupled to one of the materials used in forming the liposome. These procedures are generally well-known in the art, and no further details in this respect are deemed necessary for a complete understanding of the invention.

When the tracer passes through the membrane, the detection antibody portion binds to analyte antibody captured on the membrane as described above. The dye-loaded liposome is thereby affixed to the membrane and detection of the dye is indicative of the presence of analyte antibodies in the patient's serum. Detection of the dye is by any conventional method dependent on the nature of the dye. Thus, if the dye is a fluorescent dye, fluorescence from the membrane is detected. If the dye is an adsorbing dye, a colored spot on the membrane is detected. In the most preferred embodiment of the invention, the dye is sulforhodamine B and the development of a pink or red color on the membrane is indicative of a Lyme disease infection in the patient.

Another aspect of the invention is a kit of materials useful for performing the assay of the invention. The kit may include a solid support having affixed thereto the denatured Borrelia burgdorferi capture antigen. Another component of the kit is a tracer having a dye and a detection antibody which binds to the analyte antibody.

In a preferred kit, the solid support is a porous membrane which is mounted over an adsorbent pad in an enclosure having access to the membrane for application of assay reagents and the dye is encapsulated in a liposome conjugated to the detection antibody.

The kit may optionally contain various buffers, vials, tubes, droppers and the like useful for performing the assay, and may include known Lyme positive and negative serum samples as standards for comparison with the unknown.

The following examples are provided to further describe the invention but are not to be considered as limitative of the invention.

EXAMPLE I

Assay for Antibody to Borrelia burgdorferi

I. Materials:

A. Preparation of Native Antigen Stock

An antigen stock was prepared by the sonication and subsequent centrifugation of Borrelia burgdorferi (ATCC #B31, 35210), grown in BSK II media (ATCC #1316). The supernatent containing antigen was diluted in Dulbecco's Phosphate Buffered Saline (D-PBS) (Gibco, CAT #310-4040AC Grand Island, New York), to a final protein concentration of 5 mg/ml.

B. Preparation of Denatured Capture Antigen Stock

The native antigen stock from A (1-2 mg/ml protein) in a solution containing (final concentration) 2.5% SDS, 5% $\beta$-mercaptoethanol, 8% glycerol in 62.5 mM Tris pH 8.5 buffer was heated at 95°C for 5 minutes, diluted to a desired concentration in a buffer containing 192 mM glycine, 20% methanol and 25 mM Tris pH 8.5, and immobilized on a nitrocellulose membrane as a capture for Lyme antibody in serum.

C. Assay Device

Top Layer -

Five micron pore size nitrocellulose membrane (MSI, Westboro, Massachusetts)

Next Layer -

Non-woven rayon sheet (Schleicher and Schuell, Keene, New Hampshire #5-S).

Bottom Layer -

Cellulose adsorbent pads (2) (Filtration Sciences, Mount Springs, Pennsylvania #ED 320-200).

These layers were encased in a plastic holder which included a receiving well formed above the top layer. All reagents were added through the receiving well.

D. Serum Diluent

Reagent [PBS hemagglutination buffer (BBL #11248 Cockeysville, Maryland), 10% v/v goat serum (Gibco, Catalog number 200-61210AJ, Grand Island, New York), 0.05% w/v (polyoxyethylene sorbitol monolaureate) (TWEEN 20), 0.2% sodium azide, pH 7.2]

E. Detection reagent:

Liposomes containing encapsulated sulforhodamine B and conjugated to goat anti-human immunoglobulin in the following buffer: 50 mM 3-(N-morpholino)-2-hydroxypropane, 20 mM ethylenediaminetetraacetic acid disodium salt, 0.05% v/v dimethyl sulfoxide, 0.2% w/v sodium azide, 1.25% w/v glycerol, 0.8% bovine serum albumin (BSA), pH 7.4.

II. Assay Procedure:

The capture antigen stock (300 µl) was spotted onto the membrane in the shape of a triangle. In the center of this antigen triangle, 1 µl of human immunoglobulin G (IgG) (62.5 ng/µl) was spotted to act as a control spot. The membrane was then placed on an aspirator, and 200 µl of blocking buffer (50 mM Tris, 3% w/v BSA, 10% w/v sucrose, 0.02% w/v sodium azide, pH 8.0) was added. The membrane was then air dried at 45°C for 15 minutes, followed by a room temperature incubation in >50% relative humidity for 20 minutes.

Patient test serum (25 µl) in serum diluent (250 µl) was added dropwise through the window of the assay device onto the antigen-spotted nitrocellulose membrane. After the sample had completely soaked through the membrane, 300 µl of post-sample wash was added [50 mM Trisma-Base (Sigma), 2 M sodium chloride, 1% w/v TRITON X-705 (70% w/v solution, (Sigma), 0.2% w/v sodium azide, pH 8.0].

Detection reagent (300 µl) was added, followed by two increments of 300 µl each of post-liposome wash (PBS, 0.5% TWEEN 20, and 0.2% w/v sodium azide pH 7.2). After the wash had soaked through the membrane, the window area of the device was observed. Appearance of a red to pink triangle indicates that the patient serum is positive for antibodies against Borrelia burgdorferi. If only a small red dot is present in the center of the window in the absence of a red or pink triangle, the patient is negative for those antibodies. If no dot is seen, the test should be repeated. The total elapsed time measured from application of the diluted test serum until color formation is about 1 1/2 minutes.

EXAMPLE II

Comparison of Specificity Using Native and Denatured Antigen

Serum samples known to be Lyme negative, serum samples known to be Lyme positive and serum samples known to syphilis positive and Lyme negative were obtained from various sources (New England Medical Center, Boston, Massachusetts, Roche Biomedical Laboratory, Burlington, North Carolina and North Carolina State Laboratory, Raleigh, North Carolina) were assayed according to the procedure of Example I using native antigen as prepared in IA and denatured antigen as prepared in IB as the capture antigen. The following results were obtained where S refers to syphilis, L to Lyme pos to positive and neg to negative.

| Sample Number | Description | Native Antigen | Denatured Antigen |
|---|---|---|---|
| 1 | S pos | -/+ | - |
| 2 | S pos | - | - |
| 3 | S pos | +/- | - |
| 4 | S pos | + | - |
| 5 | S pos | - | - |
| 6 | S pos | - | - |
| 7 | S pos | - | - |
| 8 | S pos | - | - |
| 9 | S pos | -/+ | - |
| 10 | S pos | - | - |
| 11 | L neg | - | - |
| 12 | L neg | - | - |
| 13 | L neg | - | - |
| 14 | L neg | - | - |
| 15 | L pos | + | ++ |
| 16 | L pos | + | ++ |
| 17 | L pos | + | +++ |
| 18 | L pos | -/+ | +/- |
| 19 | L pos | - | +/- |
| 20 | L pos | - | - |
| 21 | L pos | -/+ | + |

| 22 | L pos | + | ++ |
| 23 | L pos | + | ++ |
| 24 | L pos | ++ | ++++ |
| 25 | L pos | ++ | ++++ |
| 26 | L pos | ++ | ++++ |
| 27 | L pos | + | +++ |

Key: +          definite positive
        +/-          faint positive
        -/+          very faint positive
        -          negative
        ++, +++, ++++          increasingly deeply colored positive spots

It is seen from the above chart that with native antigen for capture, 21 out of 27 samples were correctly identified whereas with denatured antigen, 26 out of 27 samples were correctly identified. In particular, 4 of 10 syphilis positive, Lyme negative samples gave false positives with the native antigen but were correctly identified as Lyme negative using denatured antigen for capture.

**Claims**

1. A method of assay for Lyme disease comprising:
   a) passing a serum sample suspected of containing anti-Borrelia burgdorferi antibodies through a porous membrane coated with a sonicated and denatured Borrelia burgdorferi capture antigen whereby said anti-Borrelia burgdorferi antibodies in said sample bind to said capture antigen to give a bound fraction containing anti-Borrelia burgdorferi antibodies on said membrane;
   b) contacting said bound fraction with a tracer including a detection antibody and a dye, said detection antibody binding to the anti-Borrelia burgdorferi antibody portion of said bound fraction; and
   c) determining that anti-Borrelia burgdorferi antibodies are present in said serum by detecting dye on said membrane.

2. The method of Claim 1 wherein said capture antigen has been denatured by heating.

3. The method of Claim 1 wherein said capture antigen has been denatured by a denaturant.

4. The method of Claim 1 wherein said membrane is further coated with an inert protein which fills binding sites of said membrane unoccupied by said capture antigen.

5. A method of assay for Lyme disease comprising:
   a) contacting a sample suspected of containing anti-Borrelia burgdorferi antibodies with a solid support coated with a denatured Borrelia burgdorferi capture antigen whereby said anti-Borrelia burgdorferi antibodies in said mixture bind to said capture antigen to give a bound fraction containing anti-Borrelia burgdorferi antibodies on said solid support;
   b) contacting said bound fraction with a tracer including a detection antibody and a dye, said detection antibody binding to the anti-Borrelia burgdorferi antibody portion of said bound fraction; and
   c) determining that anti-Borrelia burgdorferi antibodies are present in said sample by detecting dye on said solid support.

6. A method of assay for Lyme disease comprising:

a) passing a serum sample suspected of containing anti-Borrelia burgdorferi antibodies through a porous membrane coated with a sonicated Borrelia burgdorferi capture antigen which has been denatured with sodium dodecyl sulfate whereby said anti-Borrelia burgdorferi antibodies in said mixture bind to said capture antigen to give a bound fraction containing anti-Borrelia burgdorferi antibodies on said membrane;

b) contacting said bound fraction with a detection antibody conjugated to a liposome encapsulating an adsorbing dye, said detection antibody binding to the anti-Borrelia burgdorferi antibody portion of said bound fraction; and

c) determining that anti-Borrelia burgdorferi antibodies are present in said serum by detecting dye on said membrane.

7. A kit of materials for performing an assay for anti-Borrelia burgdorferi antibody comprising a solid support having thereon a coating of denatured Borrelia burgdorferi capture antigen and a tracer comprising a labeled detection antibody.

8. The kit of Claim 7 further comprising a coating of inert protein on said solid support to fill binding sites unoccupied by said capture antigen.

9. A kit of materials for performing an assay for anti-Borrelia burgdorferi antibody in a serum sample comprising:

a) an enclosure;

b) a filter stack in said enclosure, said filter stack including a porous membrane and a pad of adsorbent material in contact with said membrane, said membrane having thereon a coating of denatured Borrelia burgdorferi capture antigen;

c) a tracer comprising an antihuman detection antibody conjugated to a liposome encapsulating an adsorbing dye; and

d) a buffer.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| Y | US-A-4 859 419 (M.E. MARKS ET AL.) <br> * column 5, line 1-3 - column 5, line 24; claim 24 * | 1-8 | G01N33/569 <br> G01N33/543 |
| Y | JOURNAL OF CLINICAL MICROBIOLOGY <br> vol. 27, no. 8, 1 August 1989, WASHINGTON DC USA <br> pages 1854 - 1858; <br> K.E. HECHEMY ET AL.: 'Fluoroimmunoassay studies with solubilized antigens from Borrelia burgdorferi' <br> * page 1854, column 2, line 1 - page 1855, column 1, line 39 * | 1-8 | |
| A | PATENT ABSTRACTS OF JAPAN <br> vol. 14, no. 11 (P-988)(3954) 1 November 1990 <br> & JP-A-1 259 258 ( TOKUYAMA SODA COMPANY ) 16 October 1989 <br> * abstract * | 1-9 | |
| A | EP-A-0 252 641 (MINNESOTA MINING AND MANUFACTURING COMPANY) <br> * the whole document * | 1-9 | **TECHNICAL FIELDS SEARCHED (Int. Cl.5 )** <br><br> G01N |

The present search report has been drawn up for all claims

| Place of search | Date of completion of the search | Examiner |
|---|---|---|
| THE HAGUE | 23 JUNE 1992 | VAN BOHEMEN C.G. |